# EUROPEAN PATENT APPLICATION

(11) **EP 4 357 458 A1**
(43) Date of publication of application: **24.04.2024**
(21) Application number: 22202390.5
(22) Date of filing: 19.10.2022
(51) Int. Cl.: C12N 15/113, A61K 31/713, A61P 1/16, A61K 9/127

(54) **INHIBITORS FOR FRA-1 AND FRA-2 FOR USE IN THE TREATMENT OF ORGAN OR TISSUE FIBROSIS**

(71) Applicant: Universitätsmedizin der Johannes Gutenberg-Universität Mainz, 55131 Mainz (DE)
(72) Inventor: SCHUPPAN, Detlef, 55122 Mainz (DE); GIARDINO, Mariacristina, 55116 Mainz (DE); STEINBACH, Florian, 55131 Mainz (DE); BOCKAMP-VILLAMIL, Ernesto, 55126 Mainz (DE)
(74) Representative: Patentanwälte Dr. Keller, Schwertfeger Partnerschaft mbB

(57) **Abstract**

The present invention relates to inhibitors and pharmaceutical compositions for inhibiting the expression of Fos-related antigen-1 (Fra-1) or Fos-related antigen-2 (Fra-2) in fibrogenic tissue cells for use in the treatment or prevention of organ or tissue fibrosis. The inhibitor of the present invention is characterized in that it interferes with Fra-1 or Fra-2 expression, resulting in a reduction in the amount of Fra-1 or Fra-2 in said fibrogenic cells to levels found in non-fibrotic healthy cells or tissues.

## Description

The present invention relates to novel inhibitors for inhibiting the expression of Fos-related antigen-1 (Fra-1) or Fos-related antigen-2 (Fra-2) primarily in fibrogenic tissue cells and the use in the treatment of organ or tissue fibrosis, in particular liver fibrosis, pulmonary fibrosis or kidney fibrosis. The inhibitor is characterized in that it interferes with Fra-1 or Fra-2 expression, resulting in a reduction in the amount of Fra-1 or Fra-2 in said fibrogenic tissue cells to levels found in non-fibrotic healthy cells or tissues.

Fibrotic tissue remodelling in liver fibrosis or pulmonary fibrosis often results in advanced (severe) organ malfunction and is associated with a high morbidity and mortality. Organ fibrosis, especially of the liver, lungs and kidneys, is the cause of almost 50% of worldwide morbidity and mortality due to chronic diseases (*Friedmann SL. et al*., *2013; Rockey DC. et al*., *2015; Distler JHW. et al*., 2019). Currently no effective antifibrotic therapy is available in the clinic. Thus, there is a need for effective antifibrotic therapies for the treatment of liver fibrosis and pulmonary fibrosis.

The main causes of liver fibrosis and its end-stage, cirrhosis, worldwide include chronic hepatitis B virus (HBV) and hepatitis C virus (HCV) infection, alcohol abuse, biliary and autoimmune liver diseases (like autoimmune hepatitis, primary sclerosing cholangitis (PSC), primary biliary cholangitis (PBC), various congenital and genetic liver diseases, alcoholic and especially nonalcoholic steatohepatitis (NASH), whose prevalence has increased dramatically in parallel with overweight and type 2 diabetes *(Schuppan D. et al*., *2008; Younossi ZM. et al*., *2019; Schuppan D. et al., 2018).* The accumulation of excess scar tissue, subsequent to chronic injury, is due to overproduction of key molecules of the extracellular matrix, such as collagens and non-collagen proteins, and proteoglycans/glycosaminoglycans, in an attempt to repair the "wound that does not heal". While in early stages of fibrosis organ integrity and function can be maintained, fibrosis progression to liver cirrhosis usually leads to organ failure, liver cancer, a high morbidity and finally premature death. Importantly, in liver diseases that can be treated causally in many cases, such as viral hepatitis B or C, fibrosis often still progresses despite effective antiviral treatment. Moreover, once advanced fibrosis or cirrhosis have developed, fibrosis regression with improved liver function rarely occurs.

The excess synthesis and deposition of extracellular matrix proteins, synthesized mainly by activated hepatic stellate cells and myofibroblasts, replaces functional liver cells (mainly hepatocytes) by nonfunctional scar tissue and distorts the hepatic vasculature, which results in liver functional failure and/or extrahepatic complications like portal hypertension with e.g. bleeding esophageal or gastric varices, formation of ascites, coagulation disorders, hepatic encephalopathy and predisposition to septic complications. Moreover, cirrhosis increases the risk of developing a usually incurable primary hepatocellular carcinoma (HCC) by 100 to 200-fold, with a yearly incidence between 2 and 10% (*Schuppan D. et al*., *2008*).

Similar molecular and cellular mechanisms, ECM molecules and ECM-producing cells, as they occur, for instance, in liver fibrosis also drive the development of pulmonary fibrosis and fibrosis of other mesenchymal-epithelial organs such as kidneys, skin, pancreas or the gastrointestinal tract (*Friedman SL. et al*., *2013; Rockey DC. et al*., *2016*). Therefore, targeted antifibrotic therapies, i.e., therapies that interfere with fibrosis-specific mechanisms, cells or molecules, that are effective to inhibit fibrosis in liver or lung fibrosis, are also very likely to be effective in fibrosis of kidneys, skin, pancreas or the gastrointestinal tract, especially when they are directed towards these organs. That said, the cell biology, pathophysiology and the signaling pathways and molecules involved in liver, lung and kidney fibrosis share astonishing similarities. This means that most targeted antifibrotic therapies that work in one organ will likely be effective when target to another organ undergoing fibrosis.

Pulmonary fibrosis often develops in the context of environmental exposures such as certain toxins or allergens, or as an accompaniment of an (autoimmune) syndrome *(Glass DS. et al*., *2020; Renzoni EA. et al., 2021).* Common toxic and pro-inflammatory causes are exposure to asbestos, metal dusts or organic substances, radiation or medical drugs. In addition, severe microbial infections, hypoxia, hyperoxia or unknown causes (idiopathic pulmonary fibrosis) can lead to lung fibrosis. Similar to liver fibrosis, the disease is characterized by chronic inflammation and excess scar tissue ("collagen") production and deposition with consequent distortion of lung vascular and airway structures including alveolar wall thickening, all leading to impaired pulmonary gas exchange, hypoxia, hypercapnia and pulmonary hypertension. Similar to liver fibrosis, pulmonary fibrosis dramatically increases the risk of lung cancer.

Kidney fibrosis results from autoimmune diseases, such as systemic lupus or IgA nephropathy, metabolic-vascular diseases such as in diabetes or nephrotoxins *(Ruiz-Ortega M. et al*., *2020; Distler JHW. et al., 2019).* Again, chronic inflammation and/or direct cellular damage (tubular or renal epithelial damage, similar to bile ductular damage in the liver or alveolar type 2 cell damage in the lungs). This leads to mesangial or interstitial fibroblast activation (similar to hepatic stellate cells and portal fibroblast activation in the liver, or to alveolar and interstitial lung fibroblast activation in the lungs, respectively). The result is mesangial (glomerular) or interstitial kidney fibrosis, with kidney failure as end stage condition.

There is an increasing interest in developing antifibrotic programs. However, efficacy of potentially promising therapeutic approaches has not yet been proven in humans, despite a vast potential market for effective antifibrotic agents *(Friedmann SL. et al*., *2013; Schuppan D. et al*., *2018).* Moreover, current preclinical in vivo models of liver fibrosis are often not performed according to best standards and do often not replicate human chronic and fibrotic liver diseases which may result in disappointing phase 1-2 clinical trials *(Popov Y. et al*., *2009; Kim YO. et al*., *2017; Farrell G. et al., 2019).* Lack of clinical trials is due to the requirement of long follow-up studies and to the fact that liver biopsy, an invasive procedure, is still the gold-standard method for detecting changes in fibrosis. Notably, the requirement for long-term follow up in clinical phase 1-2 trials will likely not be necessary, since prediction of antifibrotic effects in liver but also in lung and kidney is now possible via the development of sensitive serum/plasma protein biomarkers of fibrosis and fibrogenesis *(Karsdal MA. et al*., *2020; Schuppan D. et al*., *2021).*

Authoritative and recent reviews have summarized the current state of the art of antifibrotic drug development for fibrosis of the liver *(Friedmann SL. et al*., *2013; Distler JHW. et al*., *2019; Ruiz-Ortega* M. *et al*., *2020; Schuppan D. et al., 2013; Schuppan D. et al., 2018),* lungs *(Friedmann SL. et al., 2013; Distler JHW. et al., 2019; Ruiz-Ortega M. et al., 2020; Schuppan D. et al., 2013; Schuppan D. et al., 2018)* and kidneys *(Friedmann SL. et al*., *2013; Distler JHW. et al*., *2019; Ruiz-Ortega M. et al., 2020; Schuppan D. et al., 2013; Schuppan D. et al., 2018).*

Fos-related antigens-1 and -2 (Fra-1 and Fra-2) belong to the activator protein 1 (AP1) family of transcription factors and are involved in a broad variety of cellular processes, such as proliferation or differentiation. Fra-1 and Fra-2 form homodimers and heterodimers as part of a complex mode of transcriptional regulation, and are induced by a large variety of cellular signals. Mice that are transgenic for Fra-2 in all cells and overexpress Fra-2 constitutively develop a pulmonary and skin fibrosis, while mice overexpressing Fra-1 in all cells and constitutively, develop biliary fibrosis resembling human primary sclerosing cholangitis (*Reich N. et al., 2010; Eferl PNAS, 2008).*

Fra-2 is the most recently discovered and least described member of the AP-1 family of transcription factors. The Fra-2 gene consists of five regions homologous to other Fos proteins as members of the AP-1 family, including the basic leucine-zipper motif responsible for dimerization, but it differs from c-fos in lacking the strong transactivation domain (*J*. *Hess et al., 2004)*. Elevated Fra-2 expression has been described in several chronic lung diseases, such as chronic obstructive pulmonary disease, asthma and pulmonary fibrosis. Several stimuli can cause transcriptional upregulation of Fra-2, including phorbol esters (e.g. TPA), increased cAMP and Ca²⁺levels (*Yoshida T. et al*., 1993) and diverse growth factors such as PDGF-BB or TGFβ *(Biasin V. et al., 2014; Reich N. et al., 2010).*

The presence of an AP-1 binding site in the promoter region of Fra-2 also suggests auto-regulatory mechanisms and cross regulation between different AP-1 family member subunits (*Yoshida T. et al*., *1993; Sonobe M.H. et al.,* 1995). Indeed, Fra-2 expression can be induced by binding of the general AP-1 dimeric transcription factor c-jun/c-fos to the Fra-2 promoter. Once Fra-2 translation is activated, Fra-2 becomes highly abundant and the c-jun/c-fos dimers are replaced by a c-jun/Fra-2 complex, which is reported to have a lower transcriptional activity compared to c-fos/c-jun (*Suzuki T. et al*., *1991*; *Sonobe M.H. et al*., *1995)*, suggesting an auto-regulatory *negative* feedback loop of Fra-2 expression. Fra-2 expression can also be influenced by epigenetic modifications, such as trimethylation of histone 3 (H3), which inhibits Fra-2 expression, whereas a lack of H3 trimethylation leads to increased Fra-2 expression (*Kramer M. et al., 2013).*

Fra-2 activity is influenced by TGFβ *(W. Tang, et al., 1998); J. Yue, et al., 2000)* and by PDGF-BB (*Reich N. et al. 2010*), or by pro-inflammatory Th2 T cell cytokines such as IL-13 (*Fichtner-Feigl S. et al., 2006).* MAP kinases induce phosphorylation of Fra-2 in vitro to a similar extent and in a similar pattern as observed in vivo (*Gruda M.C. et al*., *1994)*. Phosphorylation of Fra-2 by MAP kinases increases its DNA binding activity (*Gruda M.C. et al*., 1994; *Zoumpourlis V. et al., 2000).*

Fra-2 has a role in the regulation of cell growth and differentiation, as well as in tissue homeostasis where it appears to integrate intra- and extracellular signals. Therefore, if aberrantly expressed, Fra-2 may be involved in the development of chronic diseases, especially in organs where it is normally expressed, such as the lung *(Foletta V.C. et al*., 1994). In healthy adults, lung immunostaining identified strong Fra-2 expression in some bronchial epithelial cells, vascular smooth muscle cells and in alveolar macrophages *(Eferl R.et al., 2008; Biasin V. et al., 2014; Birnhuber A. et al., 2019; Ucero A.C. et al., 2019).* Elevated levels of Fra-2 were reported in fibrotic lungs (and skin) of patients with systemic sclerosis (SSc) (*Eferl R. et al*., *2008*; *Reich N. et al., 2010; Maurer B. et al. 2009),* interstitial lung disease and idiopathic pulmonary fibrosis (*Eferl R. et al., 2008; Ucero A.C. et al. 2019),* in the vasculature of patients with pulmonary hypertension (*Biasin V. et al. 2014),* and in pulmonary macrophages from patients with chronic obstructive pulmonary disease *(Kent L. et al. 2008).*

A recent study showed an improved phenotype in bleomycin-induced pulmonary fibrosis, a model that is frequently used to model lung fibrosis, but that also shows limited resemblance to human pulmonary fibrosis, upon treatment with a general AP-1 family inhibitor and claimed that AP-1 inhibition should be considered as a therapeutic target for the treatment of pulmonary fibrosis (*Ucero A*.*C*. *et al. 2019).* However, it remained unclear how far this effect in the bleomycin lung fibrosis model was due to specific Fra-2 inhibition.

In the cause of fibrosis research and clinical translation, the expression of Fra-1 or Fra-2 has not been addressed by a specific- and organ-targeted pharmacological treatment. This is namely due to the lack of specific small molecule inhibitors and a lack of organ- and cell-specific targeting of of Fra-1 or Fra-2. Importantly, how far Fra-1 or Fra-2 are responsible for fibrosis in a specific organ with normal regulation of these transcription factors - as opposed to the constitutively active transgenes in all cells of the body, remains unclear. Moreover, it is likely that side effects will occur, especially when all AP-1 family members are therapeutically addressed, but also when an inhibitory drug is nor organ specific, necessitating target organ (and cell) specific delivery

EP1855524B1 describes a transgenic mouse that overexpresses the transcription factor Fos-related antigen 2 (Fra-2) in all cells of the body *(Eferl PNAS, 2008).* It was concluded that it may serve as a suitable model to study fibrotic lung and kidney diseases, since their transgenic mouse resulted in a phenotype resembling human pulmonary fibrosis and skin fibrosis resembling human scleroderma. However, it remained unclear how this reflects human pathology and what pathways were involved. Moreover, Fra-2 was overexpressed in all cells of the transgenic mouse and at a constant level, which cannot be expected to be the case in human fibrosis or in induced experimental fibrosis. Moreover, the cells that would overexpress Fra-2 in naturally occurring fibrotic diseases, or would primarily dive a fibrotic response dependent on Fra-2, remain ill-defined until today.

Against this background it is the object of the present invention to provide alternative agents and pharmaceutical compositions that are suitable for the prevention or treatment of organ or tissue fibrosis, in particular for the treatment of lung fibrosis, liver fibrosis, pancreas fibrosis, or kidney fibrosis.

This object is solved by an inhibitor for Fra-1 or Fra-2 comprising the features of claim 1. Preferred embodiments of the present invention are the subject-matter of the dependent claims.

The present invention identifies a novel in vivo target that is suitable for the treatment or prevention of organ fibrosis or tissue fibrosis, in particular for the treatment or prevention of an organ or tissue fibrosis that is kidney fibrosis, pancreas fibrosis, pulmonary fibrosis, vascular vessel fibrosis, skin fibrosis, bone marrow fibrosis, or liver fibrosis. As shown by the present invention, inhibition of Fra-1 and/or Fra-2 expression is a suitable target that can be utilized in the treatment or prevention of organ or tissue fibrosis in general. More specifically, the inhibitor of the invention is a molecule that interferes with Fra-1 or Fra-2 expression, resulting in a reduction in the amount of Fra-1 or Fra-2 in said fibrogenic cells to levels found in non-fibrotic healthy cells or tissues. Preferably, the inhibitor is a molecule that specifically and effectively interferes with Fra-1 or Fra-2 transcription, Fra-1 or Fra-2 post-transcriptional mRNA processing or Fra-1 or Fra-2 mRNA translation. As such the expression levels for Fra-1 or Fra-2 may refer to any stage of protein production, i.e. on transcription level, post-transcription level, translation level, or post post translation level.

Organ fibrosis as used in the context of the present invention is characterised by a loss of cellular homeostasis and disruption of the normal tissue architecture. Typical organ fibrosis is kidney fibrosis, pancreas fibrosis, pulmonary fibrosis, vascular vessel fibrosis, skin fibrosis, bone marrow fibrosis, or liver fibrosis.

Liver fibrosis as used in the context of the present invention refers to a condition in which a damaged liver tissue is transformed into a fibrotic tissue. The disease is associated with an excessive accumulation of extracellular matrix proteins (ECMs) including collagen that occurs in most types of chronic liver diseases.

Pulmonary fibrosis as used in the context of the present invention relates to a lung disease that occurs when lung tissue becomes damaged and scarred.

Kidney fibrosis as used in the context of the present invention relates to a kidney disease that occurs when kidney tissue becomes damaged and scarred.

The present invention is based on the surprising finding that in fibrotic wild type mice, Fra-1 and Fra-2 is dominantly expressed in activated cholangiocytes, in macrophages and in activated myofibroblasts. Fra-1 or Fra-2 protein is equally overexpressed in affected fibrotic liver cells as compared to Fra-1 or Fra-2 protein found in normal, healthy cells. Similarly, the expression levels of Fra-1 or Fra-2 are significantly higher in affected fibrogenic cells than the respective expression levels in normal, healthy cells.

The inhibitors of the present invention are characterized in that they interfere with the expression apparatus of Fra-1 or Fra-2, i.e. Fra-1 or Fra-2 transcription, mRNA processing or mRNA translation, thereby reducing the amount of Fra-1 or Fra-2 protein in said fibrinogenic tissue cells to levels comparable to the expression levels of Fra-1 or Fra-2 in non-fibrotic healthy cells.

Preferably, the fibrinogenic tissue cells are selected from the group consisting of liver cells, lung cells, kidney cells and skin cells. In a preferred embodiment, the inhibitor perturbs Fra-1 or Fra-2 expression by gene silencing or protein silencing mediated by siRNA-LNPs of the present invention.

It has been found that inhibition of Fra-1 and/or Fra-2 transcription factor in target cells that overexpress Fra-1 and/or Fra-2 is associated with antifibrotic activity. As demonstrated herein, the application of an inhibitor of the present invention in vivo resulted in a down-regulation of the expression of Fra-1 or Fra-2 in the affected fibrotic cells, as exemplified in hepatic, pulmonary and renal tissue. Target cells that previously overexpressed Fra-1 or Fra-2 showed expression levels that did not significantly differ from the expression levels of Fra-1 or Fra-2 found in normal, healthy cells. Most importantly, treatments with Fra-1 and/or Fra-2 inhibitors in form of siRNA-LNPs (siRNA lipid nanoparticles) did not show any unwanted (e.g. pro-inflammatory or toxic) side effect post transfection.

A preferred inhibitor of Fra-1 or Fra-2 expression therefore comprises small interfering RNA (siRNA) operating within the RNA interference (RNAi) pathway.

Alternatively, also miRNAs can be used which are derived from regions of RNA transcripts that fold back on themselves to form short hairpins. They are usually selected from shorter regions of dsRNA and mediate silencing of genes by repression of translation.

In alternative embodiments, the inhibitor is microRNA (miRNA) comprising a nucleotide sequence that is at least partially complementary to a nucleotide sequence of Fra-1 or Fra-2 mRNA, or parts thereof.

In a further alternative embodiment, the inhibitor is an antisense oligonucleotide (ASO) comprising a nucleotide sequence that is at least partially complementary to a nucleotide sequence of Fra-1 or Fra-2 mRNA or DNA, or parts thereof.

A preferred siRNA inhibitor according to the present invention comprises a nucleotide sequence that is at least partially complementary to a nucleotide sequence of Fra-1 or Fra-2 mRNA, or parts thereof. siRNA comprising such complementary nucleotide sequences interferes with the expression of Fra-1 or Fra-2 by degrading their mRNA after transcription, thereby preventing Fra-1 or Fra-2 translation. A preferred siRNA of the present invention is a double-stranded RNA (dsRNA) having a length of preferably between 15 to 30 bp, preferably between 20 to 25 bp. In a preferred embodiment, siRNAs of the invention can be equipped with optional overhanging nucleotides and can be modified by any method known in the art to, e.g., in order to increase stability.

By using siRNA that is complementary to the Fra-1 or Fra-2 nucleotide sequence, the expression of Fra-1 or Fra-2 can be silenced in an efficient way such that the affected cells no longer overexpress Fra-1 or Fra-2 transcription factor. Preferably, the siRNAs of the present invention are synthetic siRNAs that induce RNA interference in the fibrosis inducing liver, lung, kidney or cells.

The inhibitors of the present invention may also mediate siRNA-induced post-transcriptional gene silencing by cleaving mRNA molecules encoding for Fra-1 or Fra-2 using the RNA-induced silencing complex (RISC) pathway.

Alternatively, siRNA can be expressed by using an expression vector, in order to obtain a durable knockdown or gene silencing in the affected fibrotic liver or lung cells.

In a preferred embodiment, the siRNA inhibitor for inhibiting Fra-1 or Fra-2 expression according to the invention comprises a nucleotide sequence which comprises the following nucleic acid sequences or parts thereof. The invention also covers mutants or derivatives of these sequences.

| | | |
|---|---|---|
| Sense: | 5'-GCUCACCGCAGAAGCAGUAUU-3' | (SEQ ID NO: 1) |
| Antisense: | 5'-UACUGCUUCUGCGGUGAGCUU-3' | (SEQ ID NO: 2) |

Preferred siRNA inhibitors for inhibiting Fra-1 or Fra-2 expression contain additional modifications, such as 2'-O-methyl groups in every 2^{nd} C and/or phosphorothioate at the 3' ends. Additional substitutions, such as 2'F-pyrimidines, or locked nucleotides can be introduced to enhance siRNA stability.

In another aspect of the invention, direct derivatization of siRNA (or ASOs) is possible with covalently coupled sugars that can direct the inhibitor to surface receptors of specific cells. Examples include but are not limited to mannose derivatization for targeting the mannose receptors on macrophages, or mannose-6-phosphate for targeting fibroblasts/myofibroblast/stellate cells and endothelial cells.

In order to specifically direct siRNA that is directed against Fra-1 or Fra-2 to the affected fibrosis-inducing organ or tissue cells, the siRNA is preferably complexed with a lipid-mediated nucleic acid carrier. Preferably, the lipid-mediated nucleic acid carrier is composed of lipid nanoparticles (LNPs) formulated as Lipoplex. Lipoplexes are nano-structured complexes that have been shown to be useful vehicles in the therapeutic context of the present invention in particular for the treatment or prevention of organ fibrosis, such as liver fibrosis, pulmonary fibrosis or kidney fibrosis.

Modulating the ratio of cationic lipids and nucleic acids affects the binding of the vectors with negatively charged cell surfaces. Lipoplex suspensions are known to be unstable in aqueous suspension for long-term storage, especially with respect to hydrolysis and size stability. In a preferred embodiment, the Lipoplex is therefore composed of lipidoid, cholesterol, 1,2-dioleoyl-sn-glycero-3-phosphatidylcholine (DSPC), 1,2-Dimyristoyl-rac-glycero-3-methoxypolyethylene glycol-2000 (PEG2000-DMG) and siRNA.

Preferably, the lipidoid used in the lipoplex of the present invention comprises the following structure: 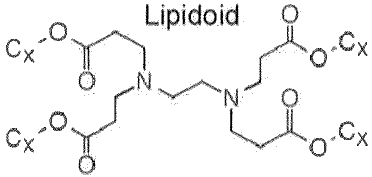 wherein Cx is selected from the group consisting of C₁₂H₂₅ (dodecyl), C₁₁H₂₃ (undecyl), C₁₃H₂₇ (tridecyl), or C₁₄H₂₉ (tetradecyl).

In alternative embodiments, higher delivery efficiency, correct particle size and zeta potential can be achieved by variations in a molecular group of said lipidoid. Examples include but are not limited to lipid nanoparticle curvature and thus size, and low in vivo toxicity or unspecific immune stimulation.

The inventive lipidoid nanoparticles mediate potent gene knockdown of Fra-1 or Fra-2 transcription factor in fibrosis-inducing (e.g. liver, lung and kidney) cells upon administration to a subject that is in need thereof.

The inventors found that siRNA-LNP mediated protein silencing in vivo can be significantly increased by altering the structural composition of the nanoparticles. Usually, the same efficiency obtained in vitro cannot be obtained in vivo. In a preferred embodiment, potent lipidoids for use in conjunction with siRNAs of the present invention are synthesized from acyl-amines with three or more substitution sites.

In a preferred embodiment, the siRNA-lipoplexes contain helper lipids such as cholesterol, DSPC and PEG2000-DMG. The choice of helper lipids did not affect the relative efficiency of the lipidoid compounds of the present invention. Lipoplex nanoparticles composed of lipidoid, cholesterol, DSPC, PEG200-DMG and siRNA against Fra-1 or Fra-2 were optimized with four aliphatic side chains and further optimized in size and zeta potential after loading with siRNAs using microfluidic or dual centrifugation technology. As further efficiency criteria, the particle size of the LNPs comprise a diameter that ranges preferably from 50 to 200 nm, preferably from 50 to 120 nm, which makes them suitable to reach the target cells in the affected liver, lung or kidney tissue. The invention also encompasses particles having a desired value within this range, or may be produced within any range within the given range of 50 to 200 nm. If the diameter of the nanoparticles should be too large, efficient delivery to a fibrotic lung, kidney tissue or liver tissue is negatively affected. Furthermore, it should be considered that lipidoids are expected to be degraded in the presence of cellular or tissue enzymes, including precursor enzymes in the blood, particularly as they raise when these LNPs are transfected in vivo.

Preferably, the siRNA-LNPs of the present invention were formulated at a lipidoid:cholesterol:DSPC:PEG molar ratio of 50:38,5:10:1,5.

In a preferred embodiment, the siRNA-LNPs of the present invention are formulated using the following ingredients and concentrations:
50% lipidoid
10% DSPC
38,5% cholesterol
1,5% DMG-PEG2000

The surface pKa of the LNPs of the invention also plays an important role in increasing in vivo efficiency. A critical pKa value averages at 5,5. For values less than 5,5, average efficiency decreased monotonically with pKa. Therefore, it is preferred that the pKa is greater or equal to 5,5. To increase efficiency, the LNPs of the present invention preferably contain one or more tertiary amines, 013 tail, or more than two tails with a pKa > 5,4 in order to achieve more than 80 % protein silencing. Preferably, the zeta potential of the inventive LNPs ranges from 0 to 100 mV, preferably > 0 to 50 mV. All intermediate values or ranges within the range of 0 to 50 mV are comprised by the present invention.

As shown herein, the siRNA loaded LNPs of the present invention result in a knockdown of Fra-1 or Fra-2 expression in the target cells when intravenously injected into fibrotic mice, thereby preventing the development of or significantly reducing liver fibrosis in vivo in models of CCI4-induced parenchymal fibrosis and in spontaneously developing secondary biliary fibrosis (Mdr2KO mice). In both models, delayed Fra-2 lipoplex treatment reduced Fra-2 transcript levels by at least 80 %, and liver fibrosis was reduced by more than 50 %.

The present invention also relates to a pharmaceutical composition, comprising an inhibitor for for inhibiting the expression of Fos-related antigen-1 (Fra-1) or Fos-related antigen-2 (Fra-2) in fibrogenic tissue for use in the treatment of organ or tissue fibrosis. In particular, the pharmaceutical composition of the present invention is suitable for the prevention or treatment of kidney fibrosis, pancreas fibrosis, pulmonary fibrosis, vascular vessel fibrosis, skin fibrosis, bone marrow fibrosis, or liver fibrosis.

The invention is explained in more detail in the following examples.

### EXAMPLES:

In the following examples, murine 3T3 fibroblasts, cholangiocytes and macrophages were transfected with increasing doses of different Fra-2 or control-luciferase siRNAs. Major fibrosis- and inflammation-related transcripts (col1a1, acta2, timp1) were determined at different time points post transfection. BALB/c mice were treated in vivo with escalating doses of CCL4 for four weeks to induce advanced liver fibrosis. One group received optimized Cy5-labeled Fra-2 siRNA-lipoplexes four times intravenously from week 3 to 4, and two control groups received luciferase-siRNA-lipoplexes or PBS. The same procedure was applied to Mdr2KO mice that develop spontaneous biliary fibrosis from age 8 to 10 weeks. Liver fibrosis was assessed by Sirius Red morphometry and hydroxyproline quantification. The expression of fibrosis and inflammation-related genes was assessed by qPCR.

Compared to mock siRNA-treated controls, the expression of col1a1, acta2 and timp1 in TGFb1-stimulated fibroblasts was downregulated 2-fold, in correlation with efficient Fra-2 knockdown. In vivo, using near infrared whole-body imaging, the Fra-2 siRNA-lipoplexes were entirely located in the liver two hours after injection. Fra2-siRNA lipoplex treatment, although being started in a later phase of fibrogenesis, resulted in significant inhibition of liver fibrosis, usually 50 %, with collagen levels close to normal controls in CCl4-induced parenchymal fibrosis, and with no further fibrosis progression in Mdr2KO mice. This was accompanied by an up to 4-fold downregulation of Col1a1 and other fibrosis related transcripts. No signs of toxicity were observed. Comparable results can be obtained with Fra-1 siRNA nanoparticles.

It therefore can be concluded that Fra-2 or Fra-1 is an attractive target for antifibrotic therapy wherein optimized Fra-1 or Fra-2-siRNA-loaded LNPs show a strong antifibrotic activity when injected in advanced phases of hepatic fibrogenesis.

### Description of the Figures:

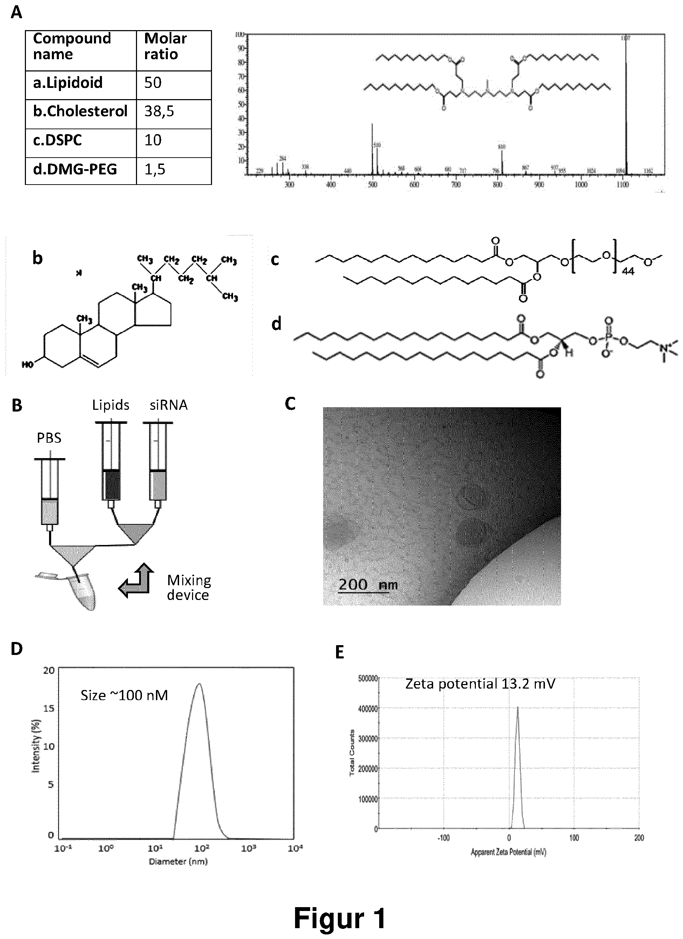

- Fig. 1: Lipoplex formulation and characterization. **A.** Lipoplex composition: a) Proprietary Lipidoid: dodecyl 3-[3-[3-[bis(3-dodecoxy-3-oxo-propyl)amino]propyl-methyl-amino]propyl-(3-dodecoxy-3-oxo-propyl)amino]propanoate (formula C67H131N3O8, M.W. [g/mol]: 1106,8); b-d) standard lipoplex components. **B.** Microfluidic technology and setup. **C.** CryoTEM characterization of siRNA-lipoplex. **D.** Dynamic light scattering analysis of lipoplex. E. zeta potential distribution (mV).

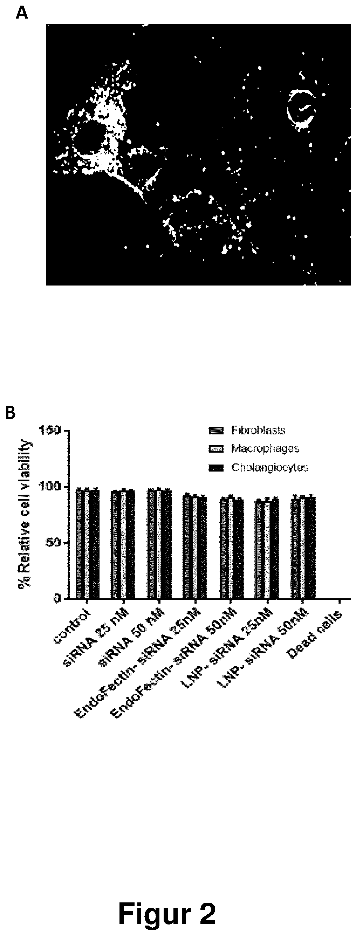

- Fig. 2: Cellular uptake and lack of toxicity of proprietary LNP-siRNA-lipoplexes in vitro. **A.** (proprietary) LNP-sFra2 uptake in 3T3 fibroblasts. Blue fluorescence: nuclei stained with DAPI; red fluorescence: LNP-Cy5-siFra2; green fluorescence: cytoplasmatic membranes stained by lipophilic carbocyanine dye DiO. **B**. Cell viability in murine 3T3 fibroblasts, 603B cholangiocytes and bone marrow derived macrophages (BMDM, M2 polarized). Controls are the non-transfected cells, the cells treated with naked 25 or 50 nM of Luc-control siRNA, and the cells treated with DMSO for 10 min (dead cells). siRNA transfection was performed with 25nM or 50nM siRNA using EndoFectin or proprietary lipoplex.

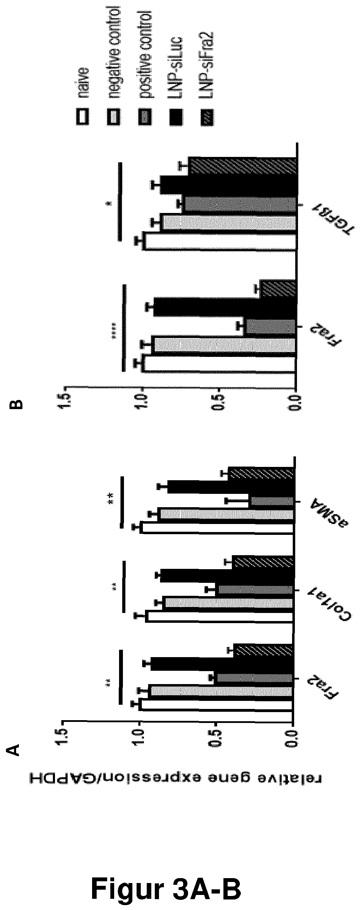

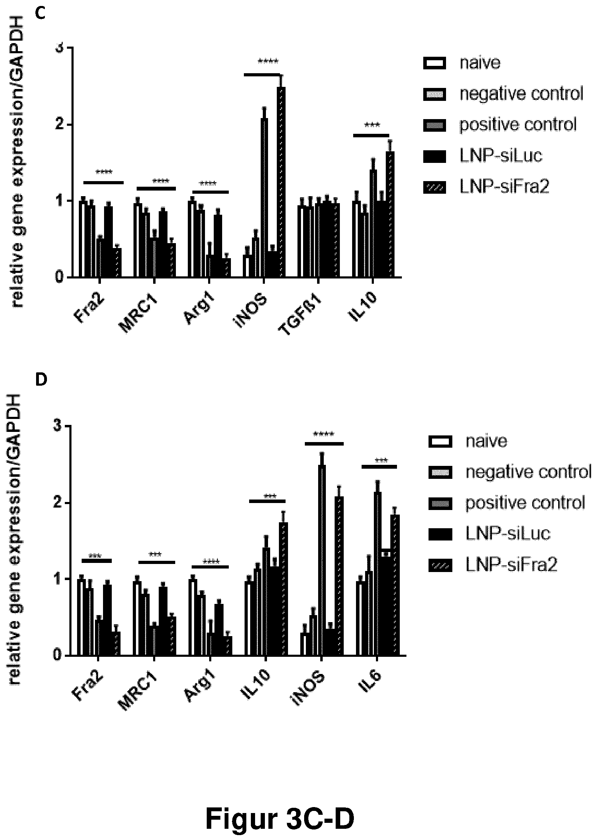

- Fig. 3A-B: LNP-siFra knockdown in 3T3 fibroblast **(A)** and 603B cholangiocytes **(B)**. Relative gene expression normalized to GAPDH transcript levels. Naive: untreated cells. Negative controls: cells transfected with either EndoFectin-siLuc or LNP-siLuc. Positive control: cells transfected with EndoFectin-siFra2. Comparisons with LNP-siFra2 transfection. Means ±SEM; n=3-5 per test. siFra2 transfection using either Endofectin or LNP-siFra2 reduce transcript levels of fra2, col1a1 and acta2 (encoding α-sma) in activated fibroblasts by 50-60%, and expression of fra2, but not of tgfb1, by 60-70% in activated cholangiocytes. ****p ≤0.0001; *p ≤0.05; **p ≤0.01 (all naive vs. LNP-siFra2).
- Fig. 3C-D: LNP-siFra2 induced knockdown in M0 **(C)** and M2 **(D)** polarized macrophages reduces M2-type and increases M1-type macrophage transcripts. Relative gene expression normalized to GAPDH transcript levels. Naive: untreated cells. Negative controls: cells transfected with either EndoFectin-siLuc or LNP-siLuc. Positive control: cells transfected with EndoFectin-siFra2. Comparisons with LNP-siFra2 transfection. M0: naive bone marrow derived macrophages (BMDM); M2: M2 polarized BMDM using IL-4 and IL-13. Means ±SEM; n=3-5 per test. C. fra2, mrc1, il6, il10: ***p ≤0.001 (naive vs. LNP-siFra2). arg1, inos: ****p ≤0.0001 (naive vs. LNP- siFra2). D. fra2, mrc1, arg1, inos: ****p ≤0.0001 (naive vs. LNP-siFra2); il10 ***p ≤0.001 (naive vs. LNP- siFra2); tgfb1: no significance.

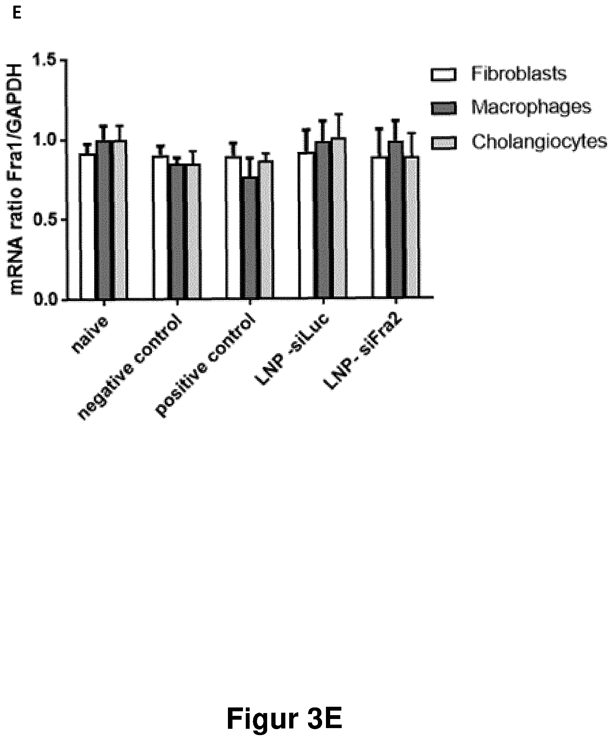

- Fig. 3E: Fra2 knockdown does not affect Fra1 expression. Relative fra1 mRNA expression in fibroblasts, macrophages and cholangiocytes after LNP-siFra2 KO. Fra1 mRNA remains unaffected.

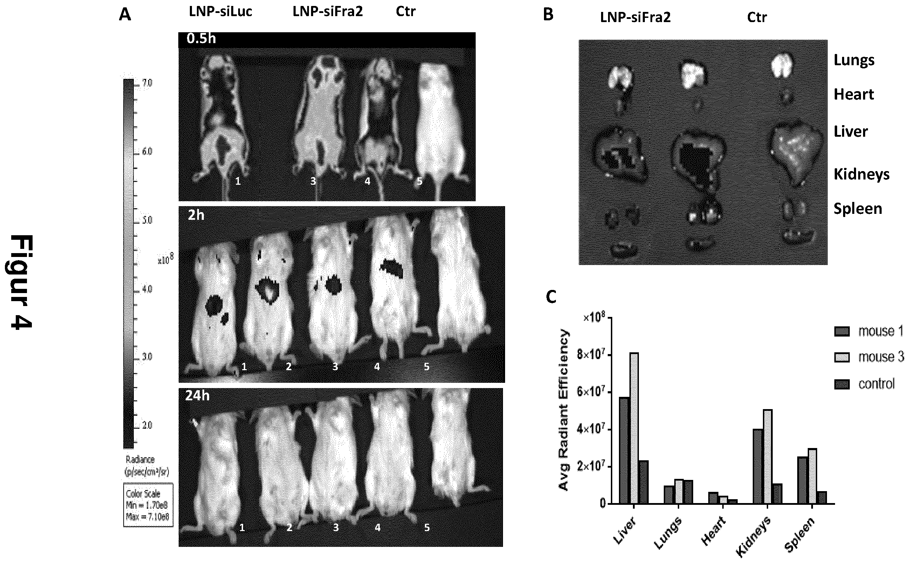

- Fig. 4: Biodistribution of LNP-siRNAs in CCl4-fibrotic mice. **A.** Near infrared (NIR) fluorescence emission after 0.5, 2 and 24 h post intravenous injection of LNP-siLuc, LNP-siFra2 and PBS (mice #1-2 injected with LNP-siLuc, mice #3-4 injected with LNP-siFra2 and mouse #5 injected with PBS). **B.** NIR fluorescence in key organs explanted 48 h after injection. **C.** Quantitative assessment of organ specific uptake from explanted organs shows major accumulation of the RNA-loaded LNPs in liver, followed by the kidneys.

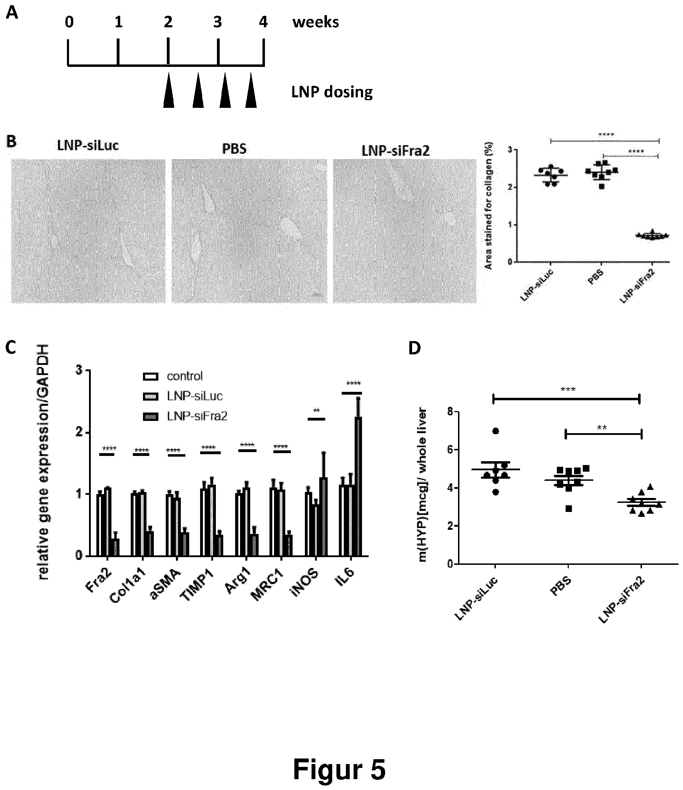

- Fig. 5: Treatment of mice with intravenous LNP-siFra2 highly significantly reduces CCl4-induced advanced liver fibrosis. **A.** Experimental scheme. Gavage with escalating doses of CCl4 gavage for 4 weeks in Balb/c mice; 4 intravenous injections of LNP-siLuc and LNP-siFra2 vs PBS in late-stage fibrosis from weeks 5-6 (n=8 per group). **B.** Sirius red staining and morphometry for hepatic collagen deposition staining shows an 80% reduction of stainable collagen in liver sections; ****p ≤0.0001 (LNP-siLuc vs. LNP- siFra2 or PBS). **C.** Quantitative RT-PCR: highly significant reduction of relative gene expression of fibrosis and M2-type macrophage related gene expression, and increase of M1-type macrophage related gene expression in livers after treatment with LNP-siFra2 vs controls. Fra2, col1a1, acta2/asma, timp1, arg1, il6; all ****p ≤0.0001; inos: ≤0.01. **D.** Hydroxyproline (biochemical collagen) content: **p ≤0.01 (LNP-siLuc vs LNP-siFra2), ***p ≤0.001 (PBS vs LNP-siFra2).

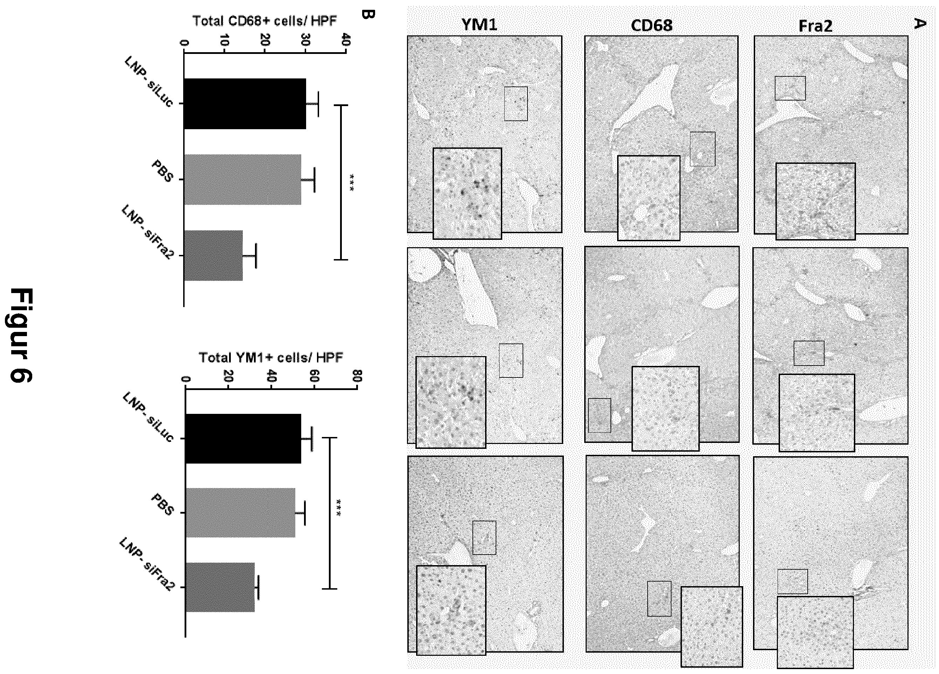

- Fig. 6: Histological expression of Fra2 and macrophage markers in CCl4-fibrotic mice with and without LNP-mediated Fra2 knockdown. **A.** Fra2 is prominently expressed in parenchymal liver cells compatible with macrophages. Fra2, CD68 (activated macrophages) and YM1 (M2-type macrophages) were all reduced in LNP-siFra2 vs control treated livers. **B.** Quantitative morphometry for CD68 and YM1 was performed on 10 random liver sections (40x magnification) per mouse and data are the means ± SD from 8 mice per group. ***p ≤0.001.

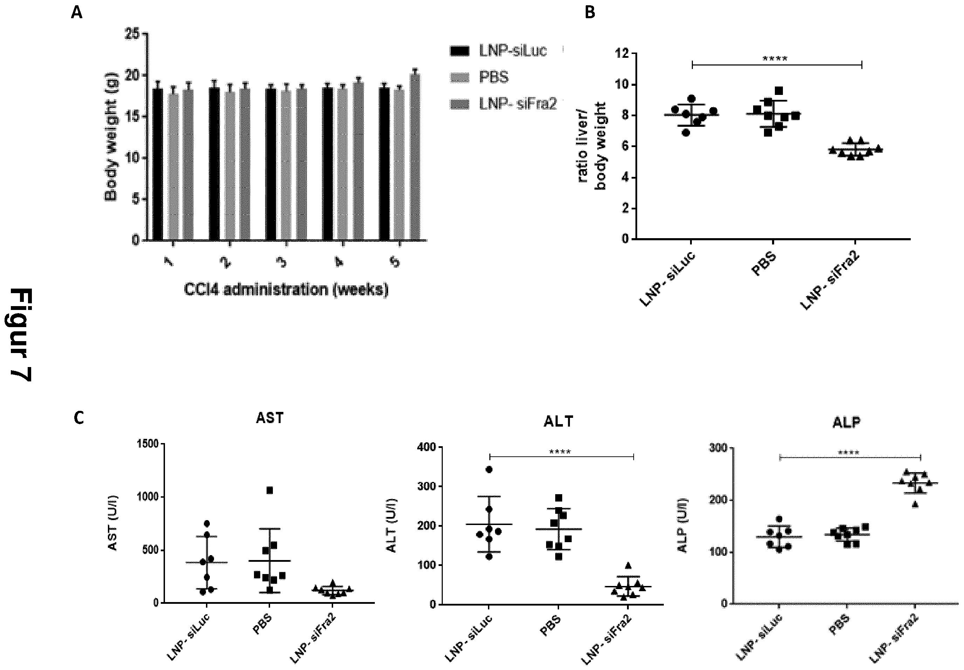

- Fig. 7: Serum ALT and liver to body weights are reduced by LNP-siFra2 vs control treatment in CCl4-fibrotic mice. **A-B.** No change in body weight with LNP-siFra2 treatment, while the liver/body weight ratio is significantly decreased in LNP-siFra2 treated CCl4-fibrotic mice. **C.** Serum levels of aspartate aminotransferase (AST) and alanine aminotransferase (ALT) are decreased, and of alkaline phosphatase (ALP) increased in LNP-siFra2 vs control treated mice (means ±SD; n=8 per group). ****p ≤0.0001.

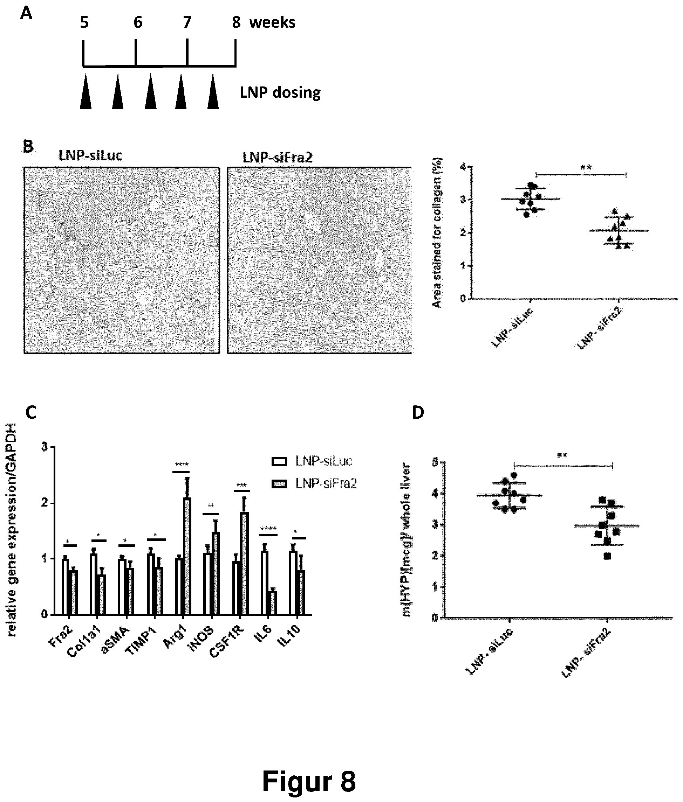

- Fig. 8: Treatment of mice with intravenous LNP-siFra2 highly significantly reduces progressive biliary fibrosis in Mdr2-/- mice. **A.** Experimental scheme. 5 intravenous injections of LNP-siLuc and LNP-siFra2 in spontaneously progressive biliary fibrosis from week 5-7 of age (n=8 per group). **B.** Sirius red staining and morphometry for hepatic collagen deposition staining shows a 35% reduction of stainable (portal tract) collagen in liver sections; **p ≤0.01 (LNP-siLuc vs. LNP-siFra2). **C.** Quantitative RT-PCR: (highly) significant reduction of relative gene expression of fibrosis and M2-type macrophage related gene expression, and increase of M1-type macrophage related gene expression in livers after treatment with LNP-siFra2 vs the LNP-siLuc control. **D.** Hydroxyproline (biochemical collagen) content. *p ≤0.05; **p ≤0.01; ****p≤0.0001.

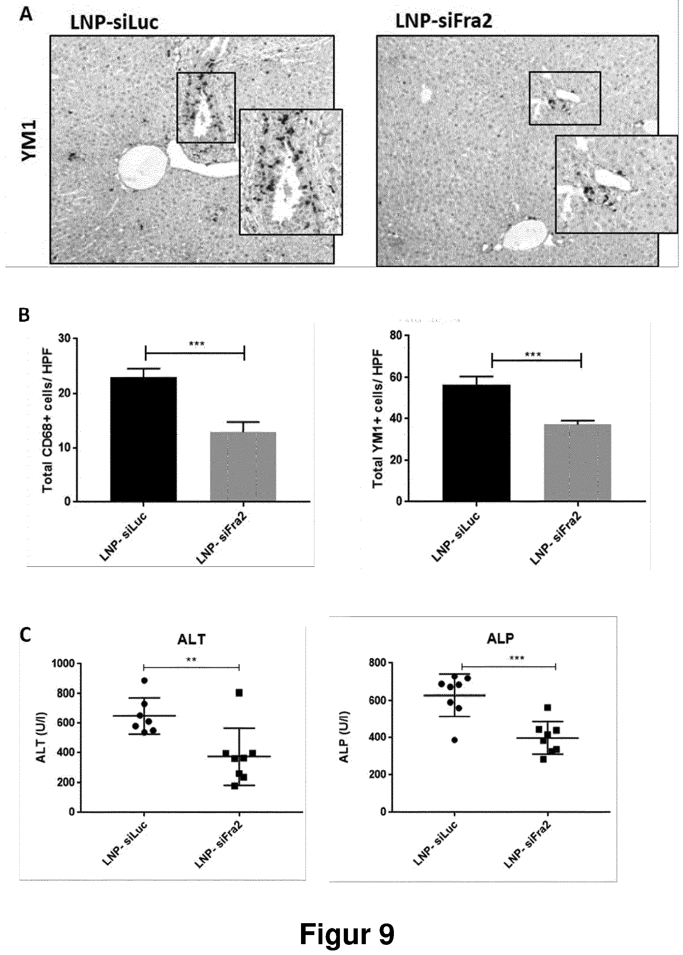

- Fig. 9: Histological expression of macrophage markers CD86 and YM1 and levels of liver enzymes in Mdr2-/- mice treated with LNP-siFra2 vs LNP-siLuc. **A.** Immunohistochemistry for YM1 expressing M2-type macrophages. **B.** Quantitative morphometry for CD68 and YM1 performed on 10 random liver sections (40x magnification) per mouse; data are the means ± SD from 8 mice per group. **C.** Serum levels of ALT and ALP are decreased, in LNP-siFra2 vs control treated mice (means ±SD; n=8 per group). For comparison: serum levels of aspartate aminotransferase (AST) and alanine aminotransferase (ALT) are decreased, and of alkaline phosphatase (ALP) increased in LNP-siFra2 vs control treated mice (means ±SD; n=8 per group). **p ≤0.01; ***p ≤0.001; ****p ≤0.0001 (not shown).

### Material and Methods

### Lipidoid synthesis and lipid compounds

The lipidoid dodecyl 3-[3-[3-[bis(3-dodecoxy-3-oxo-propyl)amino]propyl-methyl-amino]propyl-(3-dodecoxy-3-oxo- propyl)amino]propanoate (formula C67H131N3O8, M.W. [g/mol]: 1106,8), was synthesized by ChiroBlock (Wolfen, Germany) based on a screen of in silico candidates for effective siRNA delivery (33). 1,2-distearoyl-sn-glycero-3-phosphocholine (DSPC), cholesterol and 1,2-dimyristoyl-rac -glycero-3-methoxypolyethylene glycol-2000, (DMG-PEG 2000) were purchased from Avanti Polar Lipids (City, Alabama, USA).

### Formulation of lipid nanoparticles

The recipe for the lipoplex (LNP) included the lipidoid, cholesterol, DSPC and DMG-PEG 2000. The cationic lipid, cholesterol, DSPC, and DMG-PEG2000 were combined and solubilized in 90% ethanol respectively at molar ratio of cationic lipid: DSPC: cholesterol: DMG-PEG2000 of 50 : 10 : 38,5 : 1,5. The polynucleotide (PN) was dissolved at a concentration 0.4 mg/mL in 10 mM citrate, pH 3.0. To prepare LNPs, the PN (here siRNA) solution, the lipid solution and PBS buffer were injected into a microfluidic device (interdigital and caterpillar micro mixers, Fraunhofer IMM) at relative volumetric flow rates of PN: lipids: buffer of 1:1:2 using three disposable syringes, (Braun Melsungen, Germany) that were controlled by two syringe pumps (Harvard elite 11 (Fig. 1D). The two solutions (PN and lipids) were simultaneously injected into the first microfluidic channel using two syringes that were controlled by the same syringe pump with a flow rate of 5mL/min. The mixed solution was then directly injected into the second microfluidic channel, simultaneously with PBS buffer from the third syringe, using a second syringe pump with a flow rate of 10mL/min. The thus freshly prepared LNPs were dialyzed against PBS buffer using membranes or dialysis tubes (Slide-A-Lyzer^{®} MINI Dialysis 3.5K MWCO, ThermoFisher Scientific, Germany) to remove ethanol, exchange buffer and uncomplexed siRNAs.

### Dynamic light scattering measurement

Effective diameter, which describes the intensity-averaged hydrodynamic diameter of the LNPs, was determined using dynamic light scattering and used as a convenient measure of relative particle sizes. The effective diameters were calculated with NANO-flex^{®} (microtrac, particle Metrix GmbH).

### Cryo transmission electron microscopy (CryoTEM)

Sample preparation: Before grid preparation, samples were vortexed for 30s. Grids were hydrophilized by oxygen plasma (negative surface charge). Each sample was preserved in vitrified ice supported by holey carbon films on 200-mesh copper grids (QuantiFoil^{®} R2/1). Each sample was prepared by applying a 6 µL drop of sample suspension to a cleaned grid, removing buffer carefully with filter paper, and immediately proceeding with vitrification in liquid ethane at -180°C with a Leica EM GP. Grids were stored under liquid Nitrogen until being transferred to the electron microscope for imaging.

Measurement: Cryogenic TEM imaging was performed by means of a Zeiss Libra^{®} 120 under liquid N2 cryoconditions on holey carbon-coated copper grids after freezing the solution. The microscope was used at 120 kV acceleration voltage and the images were taken with a Gatan UltraScan^{®} ccd camera. Vitreous ice grids were transferred into the electron microscope using a cryostage that maintains the grids at a temperature below -170 °C.

### Determination of zeta potential

Zeta potential measurements were acquired on a Zetasizer Nano ZS (Malvern, Westborough, MA), and reported values were the average of 10-25 runs.

### Cell culture

Murine 3T3 fibroblasts and murine 603B cholangiocytes were cultured in Dulbecco's modified Eagle's medium supplemented with 10% fetal bovine serum (FBS), 1% penicillin, 1% L-glutamine, and 1% streptomycin (Sigma Aldrich) in 5% CO2 at 37 °C. The medium was changed every 2 days and the cells were separated by trypsinization using trypsin-EDTA (0.05%; LifeTechnologies, =Darmstadt, Germany) before reaching confluency. Bone marrow cells were aseptically isolated from femur and tibia bones of 6-8 weeks old C57BL/6 mice. Using a 21G needle and 1ml syringe the marrow was flushed out into IMDM (Iscove's Modified Dulbecco's Medium) plus 10% heat inactivated FBS. The cells were passed through a 100 µm cell strainer to remove cell clumps, bone, hair and tissue, and spun down at 500 × g for 5 min at 4 °C. Red blood cells were removed by adding 3-5ml of Red Blood Cell lysis buffer, incubated at RT for 10 min, cells spun down at 500xg for 5 min at 4 °C and resuspended in a petri dish containing bone marrow derived macrophage (BMDM) growth medium (3×10⁶ cells/10ml; Invivogen, CA). On day 7, formation of mature BMDM was assessed by viewing under the microscope. For M0 polarization of macrophages, only BMDM medium was used. For M2 polarization, cells were incubated in BMDM medium containing 20 ng/ml IL-4 and 20 ng/ml IL-13. Incubation ties was for 48h.

### In vitro gene knockdown

Four different siRNA sequences selected and tested for efficient Fra2 knockdown in cells using EndoFectin as in vitro transfectant (not shown). The most efficient siRNA was labelled with Cy5- near infrared (NIR) dye and complexed into the optimized lipoplex formulation for in vivo knockdown.

Cy5-labeled siFra2 (anti-Fra2 siRNA), based on the sequences:

| | |
|---|---|
| Sense: 5'-Cyanine 649 -GCUCACCGCAGAAGCAGUAUU-3' | (SEQ ID NO: 3) |
| ntisense: 5'P-UACUGCUUCUGCGGUGAGCUU-3' | (SEQ ID NO: 4) |

HPLC-purified Cy5- siRNA was purchased from Dharmacon (Cambridge, UK).

Cy5-labeled siLuc (anti-Luciferase siRNA, Biospring, Frankfurt, Germany) served as negative control:

| | |
|---|---|
| Antisense: 5'-P-UCGAAGuACUcAGCGuAAGdTsdT-3' | (SEQ ID NO: 5) |
| Sense: 5'-Cy5-cuuAcGcuGAGuAcuucGAdTsdT-3' | (SEQ ID NO: 6) |

| | |
|---|---|
| N = unmodified nucleotide, n = 2'O-methyl-modified nucleotide, s = phosphorothioate, P = phosphate | |

To assess knockdown efficiency, collagen producing murine 3T3 fibroblasts, murine cholangiocytes 603B, and M2-polarized or unpolarized BMDM were seeded in 12-well culture plates at a density of 250,000 cells per well and allowed to adhere overnight. 24 h before the knockdown 3T3 and 603B cells were preincubated with supplemented DMEM containing 5 ng/mL TGFβ1 (R&D,, Minneapolis, USA). M0 and M2 were incubated with BMDM media or 20 ng/ml IL-4 and 20 ng/ml IL-13 for 48h respectively. Afterwards the cells were incubated with siFra2 or siLuc (complexed by LNP) at a final concentration of 50 nM for 24 h at 37 °C. siFra2 and siLuc mixed with EndoFectin transfection reagent (Gene Copoeia, USA) served as positive and negative in vitro transfection controls. All experiments were performed in triplicates.

### Quantitative RT-PCR

After indicated incubation times of 3T3 fibroblasts, 603B and BMDM with LNP or EndoFectin
- siFra2 or -siLuc and after isolation of cells from homogenized harvested livers by Tissue Lyser II (Qiagen, Venlo, Netherlands), RNA was extracted, and 1 µg of total RNA was reverse-transcribed into cDNA using the qScript cDNA SuperMix (Quantas, Beverly, USA). The following TaqMan primers and probes for *Acta2, Col1a1, Gapdh, Timp1, Tgfb1* transcripts were used (Applied Biosystems, Darmstadt, Germany):

| *Acta2* | | |
|---|---|---|
| forward | 5'-ACAGCCCTCGCACCCA-3' | (SEQ ID NO: 7) |
| reverse | 5'-CAAGATCATTGCCCCTCCAGAACGC-3' | (SEQ ID NO: 8) |
| probe | 5'-GCCACCGATCCAGACAGAGT-3' | (SEQ ID NO: 9) |

| *Col1a1* | | |
|---|---|---|
| forward | 5'-ACGCATGGCCAAGAAGACA-3' | (SEQ ID NO: 10) |
| | | |
| reverse | 5'-AAGCATACCTCGGGTTTCCAC-3' | (SEQ ID NO: 11) |
| probe | 5'-AGCTGCATACACAATGGCCTAAGGGTCC-3' | (SEQ ID NO: 12) |
| | | |

| *Gapdh* | | |
|---|---|---|
| forward | 5'- CCTGCCAAGTATGATGACATCAAGA-3' | (SEQ ID NO: 13) |
| reverse | 5'- GTAGCCCAGGATGCCCTTTAGT-3' | (SEQ ID NO: 14) |
| probe | 5'-TGGTGAAGCAGGCGGCCGAG-3' | (SEQ ID NO: 15) |
| | | |

| *Timp1* | | |
|---|---|---|
| forward | 5'- TCCTCTTGTTGCTATCACTGATAGCTT-3' | (SEQ ID NO: 16) |
| reverse | 5'- CGCTGGTATAAGGTGGTCTCGTT-3' | (SEQ ID NO: 17) |
| probe | 5'- TTCTGCAACTCGGACCTGGTCATAAGG-3' | (SEQ ID NO: 18) |
| | | |

| *Tgfb1* | | |
|---|---|---|
| forward | 5'-AGAGGTCACCCGCGTGCTAA-3' | (SEQ ID NO: 19) |
| reverse | 5'-TCCCGAATGTCTGACGTATTGA-3' | (SEQ ID NO: 20) |
| probe | 5'-ACCGCAACAACGCCATCTATGAGAAAACCA-3' | (SEQ ID NO: 21) |

The following primer sequences were used for the SYBR Green methodology (Metabion, Planegg, Germany):

| *Fra1* | | |
|---|---|---|
| forward | 5'-GAGACGCGAGCGGAACAAG-3' | (SEQ ID NO: 22) |
| reverse | 5'-CTTCCAGCACCAGCTCAAGG-3' | (SEQ ID NO: 23) |
| | | |

| *Fra2* | | |
|---|---|---|
| forward | 5'-CACTCCCGGCACTTCAAAC-3' | (SEQ ID NO: 24) |
| reverse | 5'-GAGTCTGATGACTGGTCCCC-3' | (SEQ ID NO: 25) |
| | | |

| *Mrc1* | | |
|---|---|---|
| forward | 5'-AAGGCTATCCTGGTGGAAGAA-3' | (SEQ ID NO: 26) |
| reverse | 5'-AGGGAAGGGTCAGTCTGTGTT-3' | (SEQ ID NO: 27) |
| | | |

| *Arg1* | | |
|---|---|---|
| forward | 5'-CTCCAAGCCAAAGTCCTTAGAG-3' | (SEQ ID NO: 28) |
| reverse | 5'-AGGAGCTGTCATTAGGGACATC-3' | (SEQ ID NO: 29) |
| | | |

| *inos* | | |
|---|---|---|
| forward | 5'-CTATCTCCATTCTACTACTACTACCAGATCGA-3' | (SEQ ID NO: 30) |
| reverse | 5'- CCTGGGCCTCAGCTTCTCAT-3' | (SEQ ID NO: 31) |
| | | |

| *Il6* | | |
|---|---|---|
| forward | 5'-ACCAGAGGAAATTTTCAATAGGC-3' | (SEQ ID NO: 32) |
| reverse | 5'-TGATGCACTTGCAGAAAACA-3' | (SEQ ID NO: 33) |
| | | |

| *Il10* | | |
|---|---|---|
| forward | 5'-GCTCTTACTGACTGGCATGAG-3' | (SEQ ID NO: 34) |
| reverse | 5'-CGCAGCTCTAGGAGCATGTG-3' | (SEQ ID NO: 35) |

TaqMan and SYBR Green reaction mixtures were from ThermoFisher Scientific, Darmstadt, Germany).

*Gapdh* was used to normalize data (ratio of target gene to *Gapdh)* and to control for RNA integrity. The TaqMan and SYBR Green reactions were performed using a Step One Plus sequence amplification system (Applied Biosystems, Foster City, CA).

### Cell uptake

Lipoplex uptake was monitored in vitro in 3T3 murine fibroblasts. The cells were cultured as described above, fixed in 4% paraformaldehyde for 30 mins 24 h after transfection and labelled with muclear dye (5µl/mL, ThermoFisher, Germany) and NeuroDiO (PK-CA707-30021, PromoCell, Heidelberg, Germany) staining cytoplasmic membrane and intracellular membrane structures.

### Cell viability

3T3 cells were transfected for 48h using EndoFectin or lipoplex complexed with siRNAs, followed by incubation with Fixable Viability Dye eFluor780 (eBioscience) to assess viability by fluorescence-activated cell sorting (FACS Canto II, BD Bioscience, Mississauga, Canada). 10,000-50,000 cells were measured per staining, with OneComp eBeads for standardization (eBioscience) using BD FACS Diva software version 7.0. Further data analysis was carried out using open source Flowing Software 2.5.0 (Perttu Terho, Turku, Finland).

### Fibrosis models

All animal studies were approved by the local ethics committee on animal care (G 17-1-030, Government of Rhineland Palatinate, Germany). 6 weeks old female Balb/c mice were purchased from Janvier Labs (Germany) and kept under 12 h light-dark cycles at 25 °C and 40-60% humidity. Mice had access to regular chow and water ad libitum. Carbon tetrachloride (Sigma-Aldrich, St. Louis, US) diluted in mineral oil (Sigma-Aldrich, St. Louis, US) was given by oral gavage 3 times a week in an escalating dose protocol (first dose 0.875 mL/kg; 1.75 mL/kg week 1-2; 2.5 mL/kg week 3-4) for 4 weeks. Mdr2 knockout mice were 5 weeks old before treatment. At predetermined time point mice were sacrificed by cervical dislocation, blood and organs were collected for analysis.

### In vivo gene knockdown

During the third and fourth week of fibrosis induction with CCl₄, mice (n=8 per group) were anesthetized with isoflurane gas and injected intravenously 4 times (two times per week) with 1 mg/kg LNP-siFra2 control LNP-siLuc (10:1 weight-to-weight ratio LNP: siRNA; volume 50 µL), or 50 µL PBS. Five weeks old Mdr2-/- mice were injected intravenously 5 times (twice weekly in week 1-2 and once per in week 3). 48h after the last injection, organs and blood were harvested.

### Clinical chemistry

Serum was analysed for ALP (alkaline phosphatase), AST (aspartate aminotransferase), ALT (alanine aminotransferase) and creatinin by standardized assays by the clinical chemical laboratory of Mainz University Medical Center. standard laboratory test. We have checked. All creatinine values were <0.10 mg/dl, below the level of detection.

### Hydroxyproline determination

Liver collagen content was determined colorimetrically as hydroxyproline. Briefly, snap frozen liver specimens from the left and middle lobes, totalling200-300 mg, were combined, homogenized in 3 mL 6N HCl, and hydrolyzed for 16 h at 110 °C. Triplicates of 5µL were placed in a transparent 96 well-plate (Greiner bio-one, Kremsmünster, Austria), mixed with 150 µL 0.1 M citrate buffer, pH 6.0, and 100 µL containing 150 mg/mL chloramine T. After 30 min incubation at RT 100 µL of Ehrlich's reagent (1.25 g dimethyl-benzaldehyde dissolved in 100 mL distilled water) was added and incubated at 65 °C for 30 min. Absorbance was measured at 550 nm in an Infinite M200Pro spectrophotometer (TECAN, Austria). Total (µg/liver) and relative (µg/g liver) hydroxyproline content were calculated).

### Collagen (Sirius red) staining and morphometry

Formalin fixed liver sections were stained 5% Picro-Sirius red (Sigma-Aldrich, Germany) at RT for 1h and washed in distilled water and 0.5% acetic acid. 10 randomly selected fields (×40) were photographed using a Zeiss Scope A.1 microscope and an AxioCam MRC Zeiss camera (Jena, Germany). The percentage of the Sirius red-stained area was measured by ImageJ software with an adjusted threshold setting. Sections were subject to morphometry with and without portal areas, the latter representing quantitatively less but functionally more relevant collagen deposition. The means of 10 Sirius red stained areas in 10 random sections (40x magnification), 5 each from the two major liver lobes were assessed per mouse and data are the means ± SD from 8 mice per group.

### Immunohistochemistry & immunofluorescence

4 µm thick formalin fixed liver sections were boiler-treated with citrate buffer, pH 6.0, for 30 min, preincubated with 3% hydrogen peroxide for 10 min and blocked with 5% normal goat serum (Invitrogen) for 60 min at RT, followed by rat monoclonal anti mouse Fra2 (clone REY146C, Merck, MABS1261) 1/100 for 60 min at RT, followed by biotinylated goat anti-rat IgG 4°C overnight. Rabbit polyclonal anti-mouse CD68 (Abcam, ab125212) and rabbit anti-mouse Ym1 (StemCell #60130) 1/400 were followed by biotinylated goat anti-rabbit IgG 1/500). Colour was developed with Avidin-Biotin-enzyme Complex (ABC) at RT for 30 min and the DAB (3, 3'-diaminobenzidine) solution (all from Vector Labs, Burlingame, USA) for 1 min. Nuclei were counterstained with Hematoxylin (Sigma-Aldrich) for 10 seconds, followed by dehydration. Pictures were taking using a Scope A.1 microscope and an AxioCam MRC camera (both from Carl Zeiss). Morphometry was performed as outlined for Sirius red staining.

### In vivo imaging of near infrared (NIR) labeled siRNA-lipoplexes

In vivo NIR fluorescence imaging of Cy5-dye labeled LNP-siFra2 and LNP-siLuc was performed with the IVIS Spectrum Imaging system (Caliper LifeSciences, Hopkinton, US). After injection at predetermined time points, 5 random mice (mouse 1-2 treated with LNP-siLuc, mouse 3-4 treated with LNP-siFra2, and mouse 5 injected only with PBS as control) were transferred into the machine's image chamber and anesthetized temporarily with isoflurane. A picture integration time of 4 s was set for the fluorescence source. Filters were adjusted with excitation at 640 nm and emission at 700 nm to visualize Cy5-dye labeled LNP-siFra2 and LNP-siLuc.

### Ex vivo imaging of organs

48 h after the last injection of LNP-siFra2 or LNP-siLuc the mice were sacrificed and liver, spleen, lungs, heart and kidneys were immediately transferred into the imaging chamber of the IVIS Spectrum Imaging system. Image acquisition was performed with the same settings as described above. The organs of two mice (mouse 1-mouse 3) and control (only PBS) were checked.

### Statistics

Statistical significances of differences were evaluated by one-way ANOVA using GraphPad Prism version 5.0 (San Diego, USA). For statistical differences between two groups, unpaired Student's t-test was used. Data are expressed as means ± SD

### Non-Patent Literature

Biasin V., Marsh L.M., B. Egemnazarov B., Wilhelm J., B. Ghanim B., Klepetko W., et al., Meprin beta, a novel mediator of vascular remodelling underlying pulmonary hypertension, J. Pathol 233(1), 7-17 (2014)
Birnhuber A. et al., Transcription factor Fra-2 and its merging role in matrix deposition, proliferation and inflammation in chronic lung diseases; Cellular Signalling 64, (2019)
Birnhuber A., Crnkovic S., Biasin V., Marsh L.M., Odler B., Sahu-Osen A., et al.IL-1 receptor blockade skews inflammation towards Th2 in a mouse model of systemic sclerosis, Eur. Respir. J, 10.1183/13993003.00154-2019 (2019)
Distler JHW, Györfi AH, Ramanujam M, Whitfield ML, Königshoff M, Lafyatis R.Shared and distinct mechanisms of fibrosis. Nat Rev Rheumatol. 2019 Dec;15(12):705-730.
Eferl R., Hasselblatt P., Rath M., Popper H., Zenz R., Komnenovic V., et al., Development of pulmonary fibrosis through a pathway involving the transcription factor Fra-2/AP-1, Proc. Natl. Acad. Sci. U. S. A., 105 (30), 10525-10530 (2008)
Farrell G, Schattenberg JM, Leclercq I, Yeh MM, Goldin R, Teoh N, Schuppan D. Mouse Models of Nonalcoholic Steatoepatitis: Toward Optimization of Their Relevance to Human Nonalcoholic Steatohepatitis. Hepatology. 2019 May;69(5):2241-2257.
Fernando O. et al., Development of Targeted siRNA Nanocomplexes to Prevent Fibrosis in Experimental Glaucoma Filtration Surgery, Molecular Therapy, 26(12) (2018)
Fichtner-Feigl S., Strober W., Kawakami K., Puri R.K., Kitani A., IL-13 signaling through the IL-13alpha2 receptor is involved in induction of TGF-beta1 production and fibrosis, Nat. Med., 12(1), pp. 99-106 (2006)
Foletta V.C., Sonobe M.H., Suzuki T., Endo T., Iba H., Cohen D.R., Cloning and characterisation of the mouse fra-2 gene, Oncogene, 9 (11), 3305-3311 (1994)
Friedmann SL., Sheppard D., Duffield JS., Violette S., Therapie for fibrotic diseases nearing the starting line, Sci Transi Med. 2013 Jan 9; 5 (167)
Glass DS, Grossfeld D, Renna HA, Agarwala P, Spiegler P, Kasselman LJ, Glass AD, DeLeon J, Reiss AB. Idiopathic pulmonary fibrosis: Molecular mechanisms and potential treatment approaches. Respir Investig. 2020 Sep;58(5):320-335.
Gruda M.C., Kovary K., Metz R., Bravo R., Regulation of Fra-1 and Fra-2 phosphorylation differs during the cell cycle of fibroblasts and phosphorylation in vitro by MAP kinase affects DNA binding activity, Oncogene, 9 (9), 2537-2547 (1994)
Hess J., Angel P., Schorpp-Kistner M., AP-1 subunits: quarrel and harmony among siblings, J. Cell Sci. 117 (Pt 25, 5965-2973 (2004)
Karsdal MA, Daniels SJ, Holm Nielsen S, Bager C, Rasmussen DGK, Loomba R, Surabattula R, Villesen IF, Luo Y, Shevell D, Gudmann NS, Nielsen MJ, George J, Christian R, Leeming DJ, Schuppan D. Collagen biology and non-invasive biomarkers of liver fibrosis. Liver Int. 2020 Apr;40(4):736-750.
Kent L., Smyth L., Clayton C., Scott L., Cook T., Stephens R., et al., Cigarette smoke extract induced cytokine and chemokine gene expression changes in COPD macrophages Cytokine, 42 (2), 205-216 (2008)
Kim YO, Popov Y, Schuppan D. Optimized Mouse Models for Liver Fibrosis.Methods Mol Biol. 2017; 1559:279-296.
Kramer M., Dees C., Huang J., Schlottmann I., Palumbo-Zerr K., Zerr P., et al., Inhibition of H3K27 histone trimethylation activates fibroblasts and induces fibrosis, Ann. Rheum. Dis., 72 (4), 614-620 (2013)
Maurer B., Busch N., Jungel A., Pileckyte M., Gay R.E., Michel B.A., et al., Transcription factor fos-related antigen-2 induces progressive peripheral vasculopathy in mice closely resembling human systemic sclerosis Circulation, 120 (23), 2367-2376 (2009)
Popov Y, Schuppan D. Targeting liver fibrosis: strategies for development and validation of antifibrotic therapies. Hepatology. 2009 Oct;50(4):1294-306.
Poynard T. et al., Natural history of HCV infection. Baillieres Best Pract. Res. Clin. Gastroenterol. 14:211-228 (2000)
Reich N., Maurer B., Akhmetshina A., Venalis P., Dees C., Zerr P., et al., The transcription factor Fra-2 regulates the production of extracellular matrix in systemic sclerosis, Arthritis Rheum. 62(1) 280-290 (2010)
Renzoni EA, Poletti V, Mackintosh JA. Disease pathology in fibrotic interstitial lung disease: is it all about usual interstitial pneumonia? Lancet 2021 Oct 16;398(10309):1437-1449.
Rockey DC, Bell PD, Hill JA. Fibrosis--a common pathway to organ injury and failure. N Engl J Med. 2015 Mar 19;372(12):1138-49.
Ruiz-Ortega M, Rayego-Mateos S, Lamas S, Ortiz A, Rodrigues-Diez RR.Targeting the progression of chronic kidney disease. Nat Rev Nephrol. 2020 May;16(5):269-288.
Schuppan D, Afdhal NH. Liver cirrhosis. Lancet. 2008 Mar 8;371(9615):838-51.
Schuppan D, Surabattula R, Wang XY. Determinants of fibrosis progression and regression in NASH. J Hepatol. 2018 Feb;68(2):238-250.
Schuppan D, Myneni S, Surabattula R. Liquid biomarkers for fibrotic NASH - progress in a complex field. J Hepatol. 2022 Jan;76(1):5-7.
Schuppan D, Kim YO. Evolving therapies for liver fibrosis. J Clin Invest.2013 May; 123(5):1887-901.
Schuppan D, Ashfaq-Khan M, Yang AT, Kim YO. Liver fibrosis: Direct antifibrotic agents and targeted therapies. Matrix Biol. 2018 Aug;68-69:435-451.
Sonobe M.H., Yoshida T., Murakami M., Kameda T., Iba H., Fra-2 promoter can respond to serum-stimulation through AP-1 complexes, Oncogene 10(4) 689-696 (1995)
Suzuki T., Okuno H., Yoshida T., Endo T., Nishina H., Iba H., Difference in the transcriptional regulatory function between c-Fos and Fra-2, Nucleic Acids Res. 19 (20) 5537-5542 (1991)
Tang W., Yang L., Yang Y.C., Leng S.X., Elias, J.A., Transforming growth factor-beta stimulates interleukin-11 transcription via complex activating protein-1-dependent pathways, J. Biol. Chem., 273 (10), 5506-5513 (1998)
Ucero A.C., Bakiri L., Roediger B., Suzuki M., Jimenez M., Mandal P., et al., Fra-2-expressing macrophages promote lung fibrosis in mice, J. Clin. Invest., 130 (2019).
Yoshida T., Suzuki T., Sato H., Nishina H., Iba H., Analysis of fra-2 gene expression, Nucleic Acids Res. 21 (11) 2715-2721 (1993)
Yue J., Mulder K.M., Requirement of Ras/MAPK pathway activation by transforming growth factor beta for transforming growth factor beta 1 production in a Smad-dependent pathway, J. Biol. Chem., 275 (40), 30765-30773 (2000)
Younossi ZM. Non-alcoholic fatty liver disease - A global public health perspective. J Hepatol. 2019 Mar;70(3):531-544. Epub 2018 Nov 9. PMID: 30414863.
Zoumpourlis V., Papassava P., Linardopoulos S., Gillespie D., Balmain A., Pintzas A., High levels of phosphorylated c-Jun, Fra-1, Fra-2 and ATF-2 proteins correlate with malignant phenotypes in the multistage mouse skin carcinogenesis model, Oncogene, 19 (35), 4011-4021 (2000)

## Claims

1. An inhibitor for inhibiting the expression of Fos-related antigen-1 (Fra-1) or Fos-related antigen-2 (Fra-2) in fibrogenic tissue cells, for use in the treatment or prevention of organ or tissue fibrosis, wherein the inhibitor is **characterized in that** it interferes with Fra-1 or Fra-2 expression, resulting in a reduction in the amount of Fra-1 or Fra-2 in said fibrogenic cells to levels found in non-fibrotic healthy cells or tissues.

2. The inhibitor for the use according to claim 1, wherein the inhibitor interferes with Fra-1 or Fra-2 transcription, Fra-1 or Fra-2 post-transcriptional mRNA processing or Fra-1 or Fra-2 mRNA translation.

3. The inhibitor for the use according to claim 1 or claim 2, wherein the inhibitor is small interfering RNA (siRNA) comprising a nucleotide sequence that is at least partially complementary to Fra-1 mRNA or Fra-2 mRNA, or parts thereof.

4. The inhibitor for the use according to claim 1 or claim 2, wherein the inhibitor is microRNA (miRNA) comprising a nucleotide sequence that is at least partially complementary to a nucleotide sequence of Fra-1 or Fra-2 mRNA, or parts thereof.

5. The inhibitor for the use according to claim 1 or claim 2, wherein the inhibitor is an antisense oligonucleotide (ASO).

6. The inhibitor for the use according to claim 3, wherein the siRNA is complexed with a lipid-mediated nucleic acid carrier, preferably lipid nanoparticles (LNPs) formulated as lipoplex.

7. The inhibitor for the use according to claim 6, wherein the lipoplex is composed of lipidoid, cholesterol, 1,2-dioleoyl-sn-glycero-3-phosphatidylcholine (DSPC), PEG2000-DMG and siRNA.

8. The inhibitor for the use according to claim 7, wherein the lipoid comprises the following structure: 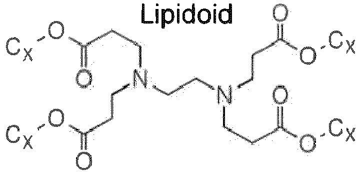 wherein Cx is selected from the group consisting of C₁₂H₂₅ (dodecyl), C₁₁H₂₃ (undecyl), C₁₃H₂₇ (tridecyl), or C₁₄H₂₉ (tetradecyl).

9. The inhibitor for the use according to any one of claims 1 to 8, wherein the diameter of the siRNA-lipoplex nanoparticles ranges from 50 to 200 nm, preferably 50 to 120 nm.

10. The inhibitor for the use according to any one of claims 1 to 9, wherein the zeta potential of the siRNA-lipoplex nanoparticles ranges from 0 to 100 mV, preferably > 0 to 50 mV.

11. The inhibitor for the use according to any one of claims 1 to 10, wherein the inhibitor of Fra-1 and/or Fra-2 expression is siRNA comprising a nucleic acid sequence as defined in SEQ ID NO: 1 or SEQ ID NO: 2.

12. The inhibitor for the use according to any one of claims 1 to 11, wherein the fibrinogenic organ or tissue cells are selected from the group consisting of liver cells, lung cells, kidney cells, pancreas cells, or skin cells.

13. The inhibitor for the use according to any one of claims 1 to 12, wherein said organ or tissue fibrosis is selected from the group consisting of kidney fibrosis, pancreas fibrosis, pulmonary fibrosis, vascular vessel fibrosis, skin fibrosis, bone marrow fibrosis, or liver fibrosis.

14. A pharmaceutical composition, comprising an inhibitor for inhibiting the expression of Fos-related antigen-1 (Fra-1) or Fos-related antigen-2 (Fra-2) in fibrogenic tissue cells according to any one of claims 1 to 13.

15. A pharmaceutical composition, comprising an inhibitor for inhibiting the expression of Fos-related antigen-1 (Fra-1) or Fos-related antigen-2 (Fra-2) in fibrogenic tissue cells according to any one of claims 1 to 13 for use in the treatment or prevention of organ or tissue fibrosis.
